# EUROPEAN PATENT APPLICATION

(11) **EP 4 616 785 A1**
(43) Date of publication of application: **17.09.2025**
(21) Application number: 24869444.0
(22) Date of filing: 01.02.2024
(51) Int. Cl.: A61B 1/317, A61B 1/00, A61B 17/94, A61B 1/012

(54) **TRANSFORAMINAL ENDOSCOPE APPARATUS WITH MOVABLE WORKING SLEEVE**

(71) Applicant: Peking University Third Hospital (Peking University Third School of Clinical Medicine), Haidian District Beijing 100191 (CN); Shanghai Reach Medical Instrument Co.,Ltd, Shanghai 201114 (CN)
(72) Inventor: LI, Weishi, Shanghai 201114 (CN); HUANG, Xiaomin, Shanghai 201114 (CN); ZHONG, Woquan, Shanghai 201114 (CN); JIANG, Shuai, Shanghai 201114 (CN); YANG, Xing, Shanghai 201114 (CN); YU, Mingpeng, Shanghai 201114 (CN)
(74) Representative: Valet Patent Services Limited
(86) International application number: PCT/CN2024/075247
(87) International publication number: WO 2025/160890

(57) **Abstract**

The invention discloses a transforaminal endoscope apparatus with a movable and positionable working sleeve, including a transforaminal endoscope part and a working sleeve connected with the transforaminal endoscope part via a positioning connecting piece by which the working sleeve moves in a horizontal direction and is detachably connected with the transforaminal endoscope part; wherein the positioning connecting piece includes a guide rod fixed on the working sleeve, a mounting base spliced with the guide rod, and a disassembly assembly disposed in the mounting base; a movable button assembly is mounted on the mounting base, and by pressing the button assembly, the mounting base is not in mutual contact with the guide rod, at the moment, the working sleeve is movable along a length direction of the guide rod; and by pressing the disassembly assembly, the mounting base is not in mutual contact with the transforaminal endoscope part, at the moment, the transforaminal endoscope part is disassembled from the working sleeve. According to the invention, a position of a transforaminal endoscope in the working sleeve is adjusted, which facilitates positioning; and the working sleeve and the transforaminal endoscope are assembled while being disassembled, which facilitates cleaning and disinfection.

## Description

### Technical Field

The invention relates to the technical field of spinal endoscopes, in particular to a transforaminal endoscope apparatus with a movable and positionable working sleeve.

### Background

An MISS (minimal invasive spine surgery) technology has been rapidly developed and has been widely applied and recognized in the industry, thereby bringing good news to millions of patients with spinal diseases around the world. The technology will lead the trend of future development in the field of spinal surgery. The MISS technology requires spinal surgeons to complete an entire surgical process by means of a surgical incision as small as possible by applying special surgical instruments and apparatuses with the assistance of imaging devices, thereby achieving the purposes of reducing the incisions, reducing tissue trauma and bleeding, improving the operation accuracy and efficacy, and promoting postoperative recovery. However, a working sleeve and a transforaminal endoscope assembly in an existing spinal endoscope cannot be assembled while being disassembled, and a position of a transforaminal endoscope in a working sleeve cannot be adjusted and positioned well during a surgery.

### Summary of the Invention

For defects existing in the prior art, the invention aims to provide a transforaminal endoscope apparatus with a movable and positionable working sleeve. A position of a transforaminal endoscope in the working sleeve is adjusted, which facilitates positioning; and the working sleeve and the transforaminal endoscope are assembled while being disassembled, which facilitates cleaning and disinfection. In order to achieve the above-mentioned objects and other advantages according to the invention, provided is a transforaminal endoscope apparatus with a movable working sleeve, including:
a transforaminal endoscope part and a working sleeve connected with the transforaminal endoscope part via a positioning connecting piece by which the working sleeve moves in a horizontal direction and is detachably connected with the transforaminal endoscope part; wherein
the positioning connecting piece includes a guide rod fixed on the working sleeve, a mounting base spliced with the guide rod, and a disassembly assembly disposed in the mounting base;
a movable button assembly is mounted on the mounting base, and by pressing the button assembly, the mounting base is not in mutual contact with the guide rod, at the moment, the working sleeve is movable along a length direction of the guide rod; and
by pressing the disassembly assembly, the mounting base is not in mutual contact with the transforaminal endoscope part, at the moment, the transforaminal endoscope part is disassembled from the working sleeve.

Preferably, the mounting base is of a U-shaped structure, a spacing block is fixedly connected to the inside of the mounting base, a through hole is formed in the middle of the spacing block, two guide rod through holes are formed in positions, close to the transforaminal endoscope part, of the mounting base, a button hole is formed in a side of one of the guide rod through holes and communicates with the guide rod through hole, and the mounting base is provided with a moving through hole in the spacing block.

Preferably, the movable button assembly includes a button plate and a rolling column vertical to and fixedly connected with the button plate, and the rolling column is located in the button hole and is in contact with the guide rod.

Preferably, the disassembly assembly includes a pressing block, a movable pointed column fixedly connected with the pressing block, a vertical moving block matched with the movable pointed column, and a transverse block fixedly connected with the vertical moving block, a mounting hole is formed in an end face of the transverse block, a spring is placed in the mounting hole, an end face of the spring is in contact with an arc-shaped end face of the mounting base, the vertical moving block is located in the moving through hole, and the pressing block is located on a side, close to the working sleeve, of the spacing block.

Preferably, a pointed end moving hole is formed in the vertical moving block, and a pointed end of the movable pointed column is of a conical structure and is in contact with an edge of the pointed end moving hole.

Preferably, an engagement piece is fixedly connected to a position, close to the vertical moving block, of the transforaminal endoscope part and is mutually engaged with the vertical moving block.

Compared with the prior art, the invention has the beneficial effects that the entire structure of the present application is simple and convenient, and by pressing the button assembly, the mounting base is not in mutual contact with the guide rod, at the moment, the working sleeve is movable along the length direction of the guide rod; by pressing the disassembly assembly, the mounting base is not in mutual contact with the transforaminal endoscope part, at the moment, the transforaminal endoscope part is disassembled from the working sleeve, which greatly facilitates the operation of a doctor during use. Moreover, by the structure, the working sleeve is stable during transfer and is convenient to disassemble and assemble, which more facilitates cleaning and disinfection.

### Brief Description of the Drawings

FIG.1 is a schematic diagram of a three-dimensional structure of a transforaminal endoscope apparatus with a movable working sleeve according to the invention;
FIG.2 is a schematic diagram of a three-dimensional exploded structure of a positioning connecting piece of a transforaminal endoscope apparatus with a movable working sleeve according to the invention;
FIG.3 is a schematic diagram of a three-dimensional structure of a mounting base of a transforaminal endoscope apparatus with a movable working sleeve according to the invention;
FIG.4 is a schematic diagram of a three-dimensional structure of a movable button assembly of a transforaminal endoscope apparatus with a movable working sleeve according to the invention;

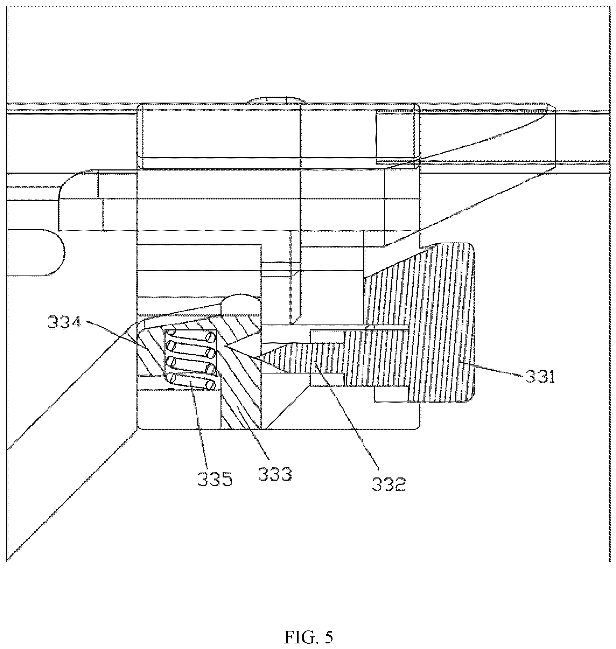

FIG.5 is a sectional view of a disassembly assembly of a transforaminal endoscope apparatus with a movable working sleeve according to the invention; and

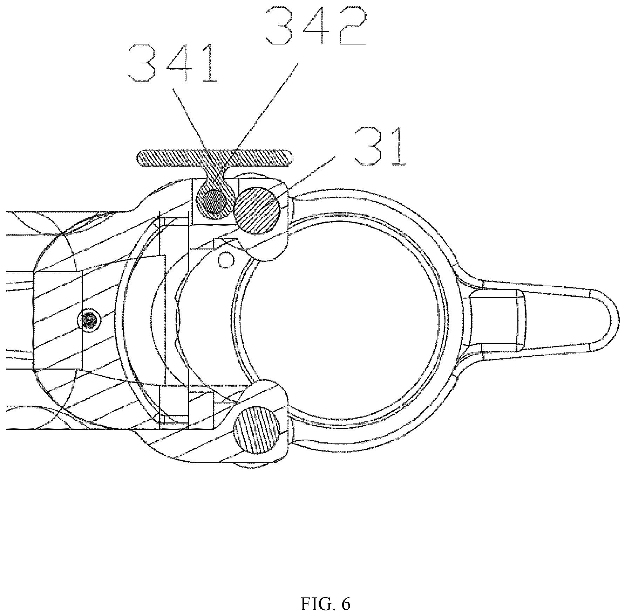

FIG.6 is a sectional view of a movable button assembly of a transforaminal endoscope apparatus with a movable working sleeve according to the invention.

### Detailed Description of the Preferred Embodiments

Technical solutions in the embodiments of the invention will be described clearly and completely below in conjunction with the accompanying drawings in the embodiments of the invention. Obviously, the described embodiments are only a part of the embodiments of the invention, not all the embodiments. Based on the embodiments in the invention, all other embodiments obtained by those of ordinary skill in the art without creative work shall fall within the protection scope of the invention.

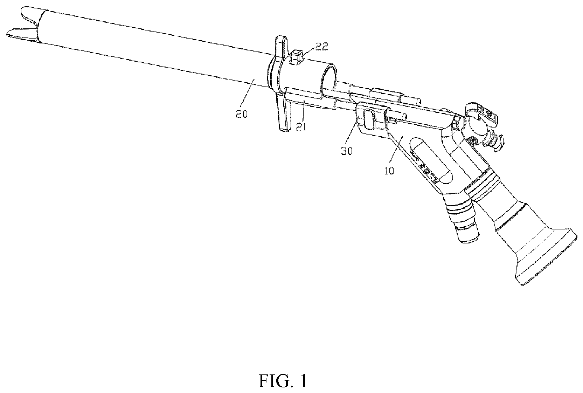

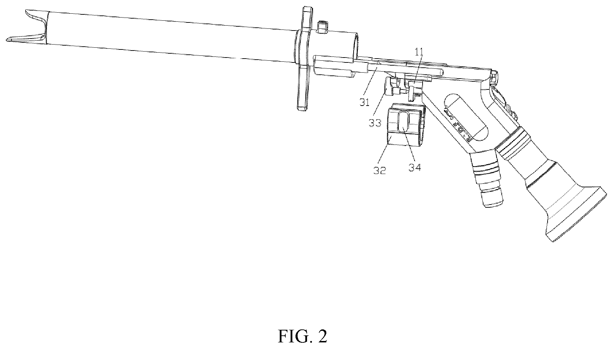

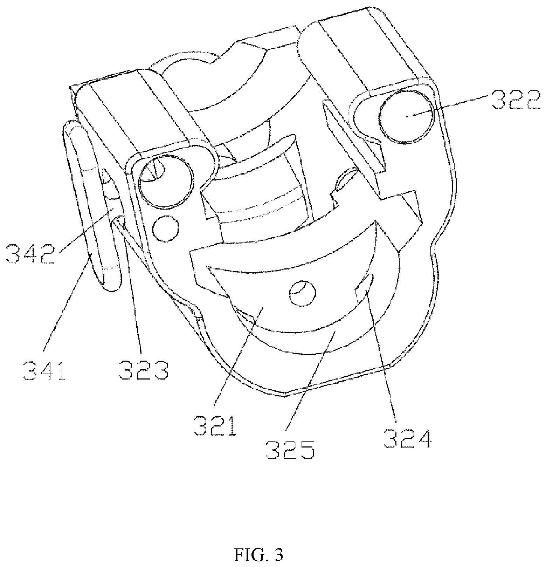

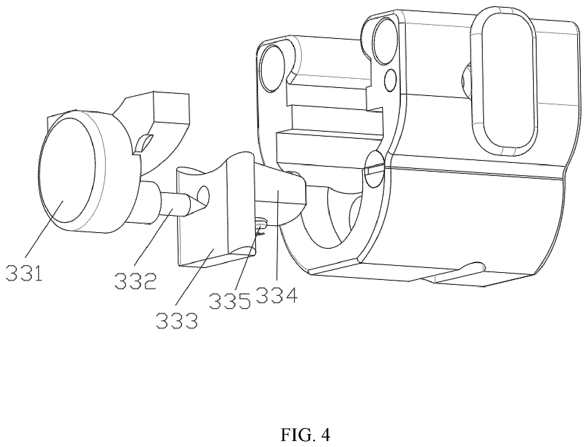

Referring to FIGS. 1 to 4, provided is a transforaminal endoscope apparatus with a movable working sleeve, including a transforaminal endoscope part 10 and a working sleeve 20 connected with the transforaminal endoscope part 10 via a positioning connecting piece 30 by which the working sleeve 20 moves in a horizontal direction and is detachably connected with the transforaminal endoscope part 10; wherein the positioning connecting piece 30 includes a guide rod 31 fixed on the working sleeve 20, a mounting base 32 spliced with the guide rod 31, and a disassembly assembly 33 disposed in the mounting base 32; a movable button assembly 34 is mounted on the mounting base 32, and by pressing the button assembly 34, the mounting base 32 is not in mutual contact with the guide rod 31, at the moment, the working sleeve 20 is movable along a length direction of the guide rod 31; and by pressing the disassembly assembly 33, the mounting base 32 is not in mutual contact with the transforaminal endoscope part 10, at the moment, the transforaminal endoscope part 10 is disassembled from the working sleeve 20. A communication sleeve 21 and a plugging/unplugging holder 22 that are symmetrically disposed are fixedly connected to a surface, close to the positioning connecting piece 30, of the working sleeve 20, the communication sleeve 21 is sleeved with the guide rod 31, and a marble, a spring placed on the marble and a bolt spliced in the spring are sequentially disposed in the plugging/unplugging holder 22. The working sleeve 20 is spliced by means of the plugging/unplugging holder 22.

Further, the mounting base 32 is of a U-shaped structure, a spacing block 321 is fixedly connected to the inside of the mounting base 32, a through hole is formed in the middle of the spacing block 321, two guide rod through holes 322 are formed in positions, close to the transforaminal endoscope part 10, of the mounting base 32, a button hole 323 is formed in a side of one of the guide rod through holes 322 and communicates with the guide rod through hole 322, and the mounting base 32 is provided with a moving through hole 324 in the spacing block 321.

Further, the movable button assembly 34 includes a button plate 341 and a rolling column 342 vertical to and fixedly connected with the button plate 341, and the rolling column 342 is located in the button hole 323 and is in contact with the guide rod 31. When a position of a transforaminal endoscope needs to be moved, by pressing the button plate 341, the rolling column 342 moves relative to the guide rod 31, when the button plate 341 is pressed in place, the rolling column 342 is not in contact with the guide rod 31, the working sleeve 20 is moved and adjusted to the required position of the transforaminal endoscope, the button plate 341 is released after position adjustment is completed, at the moment, the rolling column 342 is in mutual contact with the guide rod 31, so that the working sleeve 20 cannot move.

Further, the disassembly assembly 33 includes a pressing block 331, a movable pointed column 332 fixedly connected with the pressing block 331, a vertical moving block 333 matched with the movable pointed column 332, and a transverse block 334 fixedly connected with the vertical moving block 333, a mounting hole is formed in an end face of the transverse block 334, a spring 335 is placed in the mounting hole, an end face of the spring 335 is in contact with an arc-shaped end face of the mounting base 32, the vertical moving block 333 is located in the moving through hole 324, and the pressing block 331 is located on a side, close to the working sleeve 20, of the spacing block 321. A pointed end moving hole is formed in the vertical moving block 333, and a pointed end of the movable pointed column 332 is of a conical structure and is in contact with an edge of the pointed end moving hole. When the working sleeve 20 needs to be disassembled from the transforaminal endoscope part 10, the pressing block 331 is pressed so that the pointed end of the movable pointed column 332 moves to the inside of the pointed end moving hole; since the pointed end of the movable pointed column 332 is of the conical structure, an action force facing the moving through hole 324 will be generated on the vertical moving block 333 during the movement of the movable pointed column 332, so that the vertical moving block 333 moves along the moving through hole 324, at the moment, an upper end face of the vertical moving block 333 is separated from an engagement piece 11, and the working sleeve 20 can be disassembled from the transforaminal endoscope part 10. When the working sleeve 20 and the transforaminal endoscope part 10 are assembled, steps are the same as the above-mentioned steps, and when the pressing block 331 is released, the vertical moving block 333 automatically resets to be in contact with the engagement piece 11 under an action force of the spring 335.

Further, the engagement piece 11 is fixedly connected to a position, close to the vertical moving block 333, of the transforaminal endoscope part 10 and is mutually engaged with the vertical moving block 333.

The number of devices and the treatment scale described herein are intended to simplify the description of the invention, and the application, modification and change of the invention are obvious to the skilled in the art.

Although the embodiments of the invention have been disclosed as above, the invention is not limited to applications listed in the specification and implementations and is completely applicable to various fields suitable for the invention. For those skilled in the art, it is easy to achieve additional modifications. Therefore, when not departing from the general concept limited by the claims and an equivalent scope thereof, the invention is not limited to specific details and figures shown and described herein.

## Claims

1. A transforaminal endoscope apparatus with a movable and positionable working sleeve, **characterized by** comprising:
a transforaminal endoscope part (10) and a working sleeve (20) connected with the transforaminal endoscope part (10) via a positioning connecting piece (30) by which the working sleeve (20) moves in a horizontal direction and is detachably connected with the transforaminal endoscope part (10); wherein
the positioning connecting piece (30) comprises a guide rod (31) fixed on the working sleeve (20), a mounting base (32) spliced with the guide rod (31), and a disassembly assembly (33) disposed in the mounting base (32);
a movable button assembly (34) is mounted on the mounting base (32), and by pressing the button assembly (34), the mounting base (32) is not in mutual contact with the guide rod (31), at the moment, the working sleeve (20) is movable along a length direction of the guide rod (31); and
by pressing the disassembly assembly (33), the mounting base (32) is not in mutual contact with the transforaminal endoscope part (10), at the moment, the transforaminal endoscope part (10) is disassembled from the working sleeve (20).

2. The transforaminal endoscope apparatus with the movable and positionable working sleeve according to claim 1, **characterized in that** the mounting base (32) is of a U-shaped structure, a spacing block (321) is fixedly connected to the inside of the mounting base (32), a through hole is formed in the middle of the spacing block (321), two guide rod through holes (322) are formed in positions, close to the transforaminal endoscope part (10), of the mounting base (32), a button hole (323) is formed in a side of one of the guide rod through holes (322) and communicates with the guide rod through hole (322) in the side, and the mounting base (32) is provided with a moving through hole (324) in the spacing block (321).

3. The transforaminal endoscope apparatus with the movable and positionable working sleeve according to claim 2, **characterized in that** the movable button assembly (34) comprises a button plate (341) and a rolling column (342) vertical to and fixedly connected with the button plate (341), and the rolling column (342) is located in the button hole (323) and is in contact with the guide rod (31).

4. The transforaminal endoscope apparatus with the movable and positionable working sleeve according to claim 3, **characterized in that** the disassembly assembly (33) comprises a pressing block (331), a movable pointed column (332) fixedly connected with the pressing block (331), a vertical moving block (333) matched with the movable pointed column (332), and a transverse block (334) fixedly connected with the vertical moving block (333), a mounting hole is formed in an end face of the transverse block (334), a spring (335) is placed in the mounting hole, an end face of the spring (335) is in contact with an arc-shaped end face of the mounting base (32), the vertical moving block (333) is located in the moving through hole (324), and the pressing block (331) is located on a side, close to the working sleeve (20), of the spacing block (321).

5. The transforaminal endoscope apparatus with the movable and positionable working sleeve according to claim 4, **characterized in that** a pointed end moving hole is formed in the vertical moving block (333), and a pointed end of the movable pointed column (332) is of a conical structure and is in contact with an edge of the pointed end moving hole.

6. The transforaminal endoscope apparatus with the movable and positionable working sleeve according to claim 1, **characterized in that** an engagement piece (11) is fixedly connected to a position, close to the vertical moving block (333), of the transforaminal endoscope part (10) and is mutually engaged with the vertical moving block (333).
